# EUROPÄISCHE PATENTSCHRIFT

(11) **EP 0 609 254 B1**
(45) Veröffentlichungstag und Bekanntmachung des Hinweises auf die Patenterteilung: **19.08.1998**
(21) Anmeldenummer: 92920593.8
(22) Anmeldetag: 25.09.1992
(51) Int. Cl.: C12P 7/42

(54) **VERFAHREN ZUR HERSTELLUNG VON 2,5-DIHYDROXYPHENYLESSIGSÄURE**
METHOD OF PREPARING 2,5-DIHYDROXYPHENYLACETIC ACIDS
PROCEDE DE FABRICATION D'ACIDES 2,5-DIHYDROXYPHENYLACETIQUES

(30) Priorität: 22.10.1991 DE 4134774
(43) Veröffentlichungstag der Anmeldung: 10.08.1994
(73) Patentinhaber: BASF Aktiengesellschaft, 67063 Ludwigshafen (DE)
(72) Erfinder: STAUDENMAIER, Horst, Ralf, D-6703 Limburgerhof (DE); HAUER, Bernhard, D-6701 Fussgoenheim (DE); LADNER, Wolfgang, D-6701 Fussgoenheim (DE); MUELLER, Ursula, D-6701 Fussgoenheim (DE); PRESSLER, Uwe, D-6701 Altrip (DE); MEYER, Joachim, D-6701 Maxdorf (DE)
(86) Internationale Anmeldenummer: EP9202222
(87) Internationale Veröffentlichungsnummer: WO9308295

(56) Entgegenhaltungen:
- EP-A- 18 149
- EP-A- 89 039
- EP-A- 343 330
- Agric. Biol. Chem., Band. 49, Nr. 3, 1985 Fumiki Yoshizako et al.: "The formation of 2,6-Dihydroxyphenylacetic Acid from Phenylacetic Acid by Various Fungi ",

## Beschreibung

Die Erfindung betrifft ein neues Verfahren zur fermentativen Herstellung von Verbindungen der allgemeinen Formel I 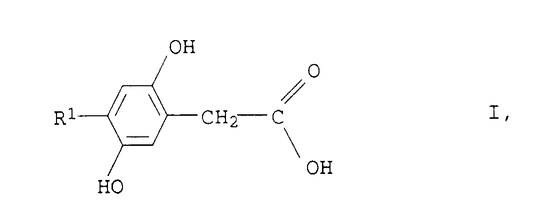 worin
- R¹: ein Wasserstoff-, ein Fluor-, ein Chlor- oder ein Bromatom bedeutet.

Verbindungen der allgemeinen Formel I sind wertvolle Zwischenprodukte bei der Farbstoff- und Pharmazeutikaherstellung.

Inbesondere die 2,5-Dihydroxyphenylessigsäure (Homogentisinsäure) wird als fotografischer Entwickler, für Kosmetikprodukte und für die Pharmazeutikaherstellung verwendet.

Es ist bekannt, daß Mikroorganismen aromatische Verbindungen hydroxylieren können. Die Hydroxylierung des aromatischen Rings stellt in vielen Fällen den ersten Schritt einer Reaktionsfolge dar, die zum Abbau der betreffenden Substanz führt.

Von Yoshizako et al. (Agric. Biol. Chem. 49 (3), 1985, 877 bis 879) ist bekannt, daß der Abbau von Phenylessigsäure bei verschiedenen Pilzen über das Zwischenprodukt 2,5-Dihydroxyphenylessigsäure (Homogentisinsäure) verläuft. Hierzu zählen beispielsweise Pilze der Gattungen Aspergillus, Fusarium, Gibberella, Mucor, Pellicularia, Penicillium, Phellinus und Rhizopus. Homogentisinsäure als Stoffwechselprodukt von Phenylessigsäure fällt jedoch nur in sehr geringen Mengen an, da sie in der Regel durch Spaltung des aromatischen Rings weiter abgebaut wird. Für die Produktion von Homogentisinsäure unter wirtschaftlichen Gesichtspunkten sind daher Wildtypstämme ungeeignet.

In der Europäischen Patentanmeldung EP 343 330 wird die Herstellung von Homogentisinsäure aus L-Tyrosin oder L-Phenylalanin mit Hilfe eines Hefestammes beschrieben. Da die dort verwendeten Ausgangssubstanzen relativ teuer sind und die Reaktion nur mit geringen Ausbeuten abläuft, ist dieser Weg wirtschaftlich unbedeutend.

In der japanischen Auslegeschrift JP-AS 6633/69 wird ein Verfahren zur Herstellung von Homogentisinsäure aus Phenylessigsäure durch aerobe Kultur einer künstlich hergestellten Variante von Penicillium chrysogenum beschrieben. Das dort in Beispiel 1 genannte Verfahren liefert Homogentisinsäure aus Phenylessigsäure in einer Ausbeute von 47 %, wobei die kumulierte Phenylessigsäurekonzentration 5 g/l betrug.

In EP 089 039 wird ein Verfahren zur Herstellung von D-β-Hydroxyalkansäuren aus Alkansäuren durch Mutanten von Mikroorganismen beschrieben, die nur eine geringe oder keine Fähigkeit zum Abbau der D-β-Hydroxyalkansäuren besitzen.

Für ein wirtschaftliches Verfahren ist einerseits eine hohe Ausbeute und andererseits eine möglichst hohe Substratkonzentration im Medium erforderlich.

Es bestand daher die Aufgabe, ein fermentatives Herstellungsverfahren für Verbindungen der Formel I bereitzustellen, das gute Ausbeuten liefert und hohe Substratkonzentrationen ermöglicht.

Demgemäß wurde gefunden, daß das eingangs definierte Verfahren zur fermentativen Herstellung von Verbindungen der allgemeinen Formel I 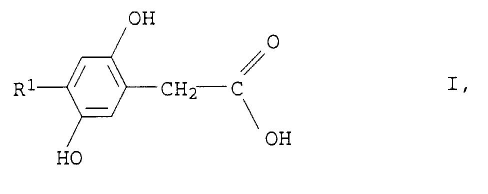 worin
- R¹: ein Wasserstoff-, ein Fluor-, ein Chlor- oder ein Bromatom bedeutet,
besonders gute Ausbeuten liefert, wenn man Verbindungen der allgemeinen Formel II 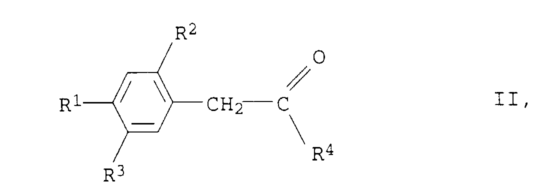 worin
- R¹: die obengenannten Reste,
- R², R³: unabhängig voneinander ein Wasserstoffatom oder eine Hydroxylgruppe oder eine Methoxygruppe und
- R⁴: eine Hydroxylgruppe oder eine Methoxygruppe oder eine Aminogruppe bedeuten,
in Gegenwart eines Mikroorganismus der Gattung Beauveria hydroxyliert, der Verbindungen der Formel II hydroxylieren, jedoch nicht als C-Quelle verwerten kann.

Die für das erfindungsgemäße Verfahren benötigten Ausgangsverbindungen der allgemeinen Formel II sind bekannt.

Sie sind beispielsweise nach in Houben-Weyl, "Methoden der organischen Chemie", Band 8 beschriebenen Verfahren herstellbar.

Die Umwandlung der oben genannten Ausgangsverbindungen zu Verbindungen der allgemeinen Formel I durch Mikroorganismen beinhaltet eine Hydroxylierung des aromatischen Kerns in der 2- und 5-Position. Wenn an diesen Positionen in der Ausgangsverbindung ein Wasserstoffatom oder eine Methoxygruppe vorliegt, so wird dieser Rest im erfindungsgemäßen Verfahren durch eine Hydroxylgruppe ersetzt.

Reste an der 4-Position des aromatischen Kerns wie ein Wasserstoff- oder ein Halogenatom werden durch das erfindungsgemäße Verfahren nicht verändert. Bevorzugt werden Ausgangsverbindungen verwendet, die an der 4-Position ein Wasserstoffatom tragen.

Neben der Hydroxylierung am Aromaten kann auch eine Veränderung an der Carboxylgruppe durch das erfindungsgemäße Verfahren stattfinden. So wird beispielsweise ein Methylester oder ein Säureamid zur freien Säure bzw. deren Salz umgewandelt. Wird als Ausgangsverbindung eine freie Säure bzw. deren Salz verwendet, so wird diese Gruppe nicht durch das Verfahren verändert.

Die für das erfindungsgemäße Verfahren geeigneten Mikroorganismen müssen einerseits die Fähigkeit besitzen, die Ausgangsverbindungen am aromatischen Kern hydroxylieren zu können, andererseits dürfen sie die Ausgangsverbindung nicht als Kohlenstoffquelle benutzen.

Solche Mikroorganismen erhält man zweckmäßigerweise durch Mutationen von Wildtypstämmen, die die Fähigkeit zur Aromatenhydroxylierung besitzen.

Bevorzugt werden als Mikroorganismen solche der Gattung Beauveria, beispielsweise Beauveria bassiana, B. densa, B. brogniartii, B. amorpha, B. vermiconia, verwendet. Besonders bevorzugt sind solche der Art Beauveria bassiana DSM 6650.

Ob ein Mikroorganismus geeignet ist, die Ausgangsverbindung in gewünschter Weise am aromatischen Kern zu hydroxylieren, läßt sich leicht mit Hilfe analytischer Methoden, z.B. der Gaschromatographie, anhand des Nährmediums bestimmen.

Zweckmäßigerweise setzt man dem Nährmedium die zu hydroxylierende Ausgangsverbindung zu und überprüft im Laufe der Züchtung, ob die Ausgangsverbindung abnimmt und die gewünschte hydroxylierte Verbindung als Metabolit auftritt. Zur Identifizierung von Metaboliten können übliche Verfahren wie "resting-cell"-Experimente, Einsatz von Hemmstoffen oder Isotopenmarkierung verwendet werden.

Von den geeigneten Mikroorganismen werden zweckmäßigerweise Mutanten erzeugt, die nicht mehr in der Lage sind, die Ausgangsverbindung als Kohlenstoffquelle zu verwenden.

Zur Erzeugung solcher Mutanten können bekannte mikrobiologische Techniken eingesetzt werden. Zur Auslösung von Mutationen können alle gängigen Methoden verwendet werden wie die Anwendung von mutagenen Substanzen, z.B. Nitrosoguanidin, Ethylmethansulfonat, Natriumnitrit, oder die Einwirkung von elektromagnetischer Strahlung wie UV-, Gamma- oder Röntgenstrahlung. Weiterhin können zur Mutagenese auch transponierbare genetische Elemente verwendet werden. Zur Isolierung der Mutanten können verschiedene Eigenschaften herangezogen werden, wie die Unfähigkeit, auf Phenylessigsäure als einziger C-Quelle zu wachsen oder die optisch erkennbare Braunfärbung infolge der gebildeten Homogentisinsäure. Gegebenenfalls kann an dieser Stelle auch noch eine Anreicherung der gesuchten Mutanten durchgeführt werden.

Das erfindungsgemäße Verfahren wird mit geeigneten Mikroorganismen durchgeführt, die in einem Nährmedium, das die Ausgangsverbindung in einer Konzentration von 1 bis 20 g/l, bevorzugt von 5 bis 15 g/l enthält, gezüchtet werden.

Bevorzugt wird das erfindungsgemäße Verfahren so ausgeführt, daß verbrauchtes Substrat durch Zudosieren einer hochkonzentrierten Substratstammlösung wieder ersetzt wird. Dadurch ist es möglich, kumulierte Substratkonzentrationen von bis zu 50 g/l im Fermentationsmedium zu erreichen.

Die Züchtungsdauer hängt von der Ausgangsverbindung und dem Mikroorganismus ab; sie beträgt in der Regel einige Tage. Zweckmäßigerweise wird die Züchtung so lange fortgesetzt, bis ein nahezu quantitativer Umsatz der Ausgangsverbindung erfolgt ist.

Die Züchtung kann in einem kontinuierlichen oder einem diskontinuierlichen Verfahren betrieben werden; bevorzugt wird jedoch eine diskontinuierliche Verfahrensweise.

Die Isolierung und Reinigung der hydroxylierten Phenylessigsäure aus dem Nährmedium kann nach bekannten Methoden erfolgen. Zweckmäßigerweise trennt man die feste Biomasse vom Nährmedium ab, extrahiert den Wertstoff z.B. mit einem organischen Lösungsmittel und isoliert den Wertstoff aus der extrahierten Phase, gegebenenfalls nach weiterer Reinigung, z.B. durch Kristallisation.

Die Erfindung wird durch die folgenden Beispiele weiter veranschaulicht.

### Beispiel 1

### Herstellung einer Mutante (LU 6577) aus Beauveria bassiana

Der Pilz Beauveria bassiana DSM 6650 wurde mit Hilfe von N-Methyl-N'-nitro-N-nitrosoguanidin (MNNG) mutagenisiert. Eine Sporensuspension des Pilzes in 0,1 M Phosphatpuffer pH 7,0, 0,1 Gew.-% Polyethoxysorbitanoleat (Tween 80®) wurde auf einen Titer von 10⁷ Sporen pro ml eingestellt. 10 ml dieser Sporensuspension wurden durch Zugabe einer Stammlösung von 5 mg/ml MNNG (gelöst in Dimethylformamid) auf eine Konzentration von 0,2 mg/ml MNNG eingestellt und 15 min unter leichtem Schütteln bei 30°C inkubiert. Dann wurden die Sporen durch Zentrifugation (5 Minuten mit 5000 Upm) geerntet und zweimal mit 10 ml Tweenpuffer gewaschen. Die Sporen wurden in Tweenpuffer aufgenommen, verdünnt und auf Komplexmedium ausplattiert. Der Vergleich mit unbehandelten Sporen ergab bei mehreren Experimenten regelmäßig eine Überlebensrate der mutagenisierten Sporen von 1 bis 2 %.

Die mutagenisierten Sporen wurden auf Agarplatten mit Komplexmedium der folgenden Zusammensetzung ausplattiert und 5 Tage bei 30°C inkubiert:

| | |
|---|---|
| 50 g/l | D-Glucose |
| 10 g/l | Hefeextrakt |
| 3,6 g/l | K₂HPO₄ |
| 1,5 g/l | KH₂PO₄ |
| 0,5 g/l | MgSO₄ x 7 H₂O |
| 0,05 g/l | MnSO₄ x H₂O |
| 2 ml/l | Spurenelement lösung |
| 20 g/l | Agar |

| Spurenelementlösung: | |
|---|---|
| 200 mg/l | Eisen(II)sulfat-1-hydrat |
| 10 mg/l | Zink(II)sulfat-4-hydrat |
| 3 mg/l | Manganchlorid-4-hydrat |
| 30 mg/l | Borsäure |
| 20 mg/l | Cobalt(II)chlorid-6-hydrat |
| 1mg/l | Kupfer(II)chlorid-2-hydrat |
| 2 mg/l | Nickel(II)chlorid-6-hydrat |
| 3 mg/l | Natriummolybdat-2-hydrat |
| 500 mg/l | Ethylendiamintetraessigsäure (EDTA) |

Von den entstandenen Einzelkolonien wurde mit Hilfe von Zahnstochern ein kleines Mycelstück in Reagenzgläser mit je 2 ml des folgenden Minimalmediums mit Phenylessigsäure als einziger Kohlenstoffquelle angeimpft:

| | |
|---|---|
| 5 g/l | Phenylessigsäure |
| 5 g/l | (NH₄)₂SO₄ |
| 3,6 g/l | K₂HPO₄ |
| 1,5 g/l | KH₂PO₄ |
| 0,5 g/l | MgSO₄ x 7 H₂O |
| 0,05 g/l | MnSO₄ x H₂O |
| 2 ml/l | Spurenelementlösung |

Die Ansätze wurden 7 Tage bei 30°C geschüttelt (180 Upm). Klone, die während dieses Zeitraums das Medium nicht dicht bewachsen hatten, wurden weiter charakterisiert. Die Anteile der nicht gewachsenen Klone betrug bei verschiedenen Experimenten zwischen 3 und 8 %.

Die weiter zu charakterisierenden Klone wurden aus dem Rückstellmuster in je 2 ml Komplexmedium mit 2 g/l Phenylesslgsäure angeimpft und 10 Tage bei 30°C schüttelnd gezüchtet.

Dann wurden die Kulturen abzentrifugiert und mit dem Kulturüberstand ein photometrischer Test durchgeführt: Zu 0,5 ml Kulturüberstand wurden 0,5 ml 1,6 % NaNO₂ und 0,2 ml 1 M H₂SO₄ gegeben und 10 min inkubiert. Dann wurden 0,25 ml 0,2 Gew.-% EDTA in 2,3 M NaOH zugegeben und die Proben bei 450 nm gegen den Leerwert gemessen. Für den Leerwert wurde bei sonst gleichem Vorgehen anstatt NaNO₂-Lösung Wasser zugegeben.

Klone, die im photometrischen Test eine Extinktion A₄₅₀ > 1,8 hatten (1-5 % der getesteten Klone), wurden in Schüttelkolbenexperimenten weiter untersucht. Dazu wurde jeweils eine Vorkultur der Klone in Komplexmedium mit 2,5 g/l Phenylessigsäure angesetzt (30 ml in 250 ml-Erlenmeyerkolben). Nach 3 Tagen Schütteln bei 30°C wurde mit je 5 ml der Vorkultur eine Hauptkultur mit 10 g/l Phenylessigsäure angeimpft und bei 30°C geschüttelt. Nach jeweils 3 und 7 Tagen wurde eine Probe gezogen und der Gehalt an Phenylessigsäure und deren Derivaten gaschromatographisch analysiert.

Es wurde 1 ml der Kulturen abgenommen, mit 100 µl 5 M HCl und 800 µl Essigester versetzt, 15 s gemischt und 2 min bei 12000 g zentrifugiert. 50 µl der organischen Phase wurden abgenommen und mit 50 µl N-Methyl-N-(trimethylsilyl)-trifluoracetamid (MSTFA) versetzt.

Die Proben wurden gaschromatographisch aufgetrennt (165°C isotherm, Säule: Methylsilicon 12,5 m, Hewlett-Packard, 1 µl Injektionsvolumen). Phenylessigsäure erschien im Chromatogramm nach 1,7 min, Homogentisinsäure nach 7,8 min.

Die Mutante Lu 6577 zeigte in diesem Test nach 7 Tagen vollständigen Umsatz von Phenylessigsäure zu Homogentisinsäure.

### Beispiel 2

### Herstellung von Homogentisinsäure aus Phenylessigsäure

Die Mutante Lu 6577 wurde in 5 x 30ml des folgenden Mediums in 250 ml-Erlenmeyerkolben angeimpft und 3 Tage bei 30°C mit 180 Upm aerob schüttelnd inkubiert:

| | |
|---|---|
| 2,5 g/l | Phenylessigsäure |
| 50 g/l | D-Glucose |
| 10 g/l | Hefeextrakt |
| 0,5 g/l | MgSO₄ x 7 H₂O |
| 1,5 g/l | KH₂PO₄ |
| 3,6 g/l | K₂HPO₄ |
| 2 ml/l | Spurenelementlösung |

Mit dieser Vorkultur wurde ein 1-l-Fermenter mit demselben Medium und einer Phenylessigsäurekonzentration von 10 g/l angeimpft. Der Fermenter wurde mit 600 Upm gerührt und mit 1 Volumen Luft pro Volumen Reaktor pro Minute begast. Immer wenn die Umsetzung soweit fortgeschritten war, daß die Phenylessigsäurekonzentration auf einen Wert zwischen 0,5 und 5 g/l abgesunken war, wurde mehrfach Phenylessigsäure in Form einer 20%igen Stammlösung in Wasser zudosiert, so daß jeweils wieder eine Konzentration zwischen 5 und 10 g/l an Phenylessigsäure im Medium erreicht wurde. In der Summe wurden auf diese Weise 30 g/l Phenylessigsäure umgesetzt. Nach Erreichen dieser Menge wurde die Kultur ohne weitere Zudosierung von Phenylessigsäure weiterinkubiert. Als die Phenylessigsäure vollständig zu Homogentisinsäure umgesetzt war, wurden die Zellen abzentrifugiert. Der Kulturüberstand wurde mit HCl auf pH = 2 gestellt und die gebildete Homogentisinsäure durch Extraktion mit Essigsäureethylester gewonnen. Nach dem Abdampfen des Lösungsmittels wurden 26,8 g Homogentisinsäure erhalten.

### Beispiel 3

### Herstellung von Homogentisinsäure aus 2-Hydroxyphenylessigsäure

Es wurde eine Vorkultur (30 ml) des Stammes Lu 6577 wie in Beispiel 2, jedoch ohne Phenylessigsäure angesetzt. Mit 5 ml der Vorkultur wurde nach 3 Tagen eine Hauptkultur (30 ml) im gleichen Medium mit 5 g/l 2-Hydroxyphenylessigsäure angeimpft. Nach 4 Tagen Schütteln bei 180 Upm und 30°C war die 2-Hydroxyphenylessigsäure vollständig zu Homogentisinsäure umgesetzt.

### Beispiel 4

### Herstellung von Homogentisinsäure aus 3-Hydroxyphenylessigsäure

In gleicher Weise wie in Beispiel 3 wurde eine Hauptkultur von Lu 6577 angesetzt, mit dem Unterschied, daß als Substrat 1 g/l 3-Hydroxyphenylessigsäure zugegeben wurde. Nach 4 Tagen schüttelnder Inkubation bei 30°C war die 3-Hydroxyphenylessigsäure zu Homogentisinsäure umgesetzt.

### Beispiel 5

### Herstellung von Homogentisinsäure aus 3-Methoxyphenylessigsäure

Wie in Beispiel 3 wurde eine Hauptkultur mit Lu 6577 angeimpft. Als Substrat wurde 1 g/l 3-Methoxyphenylessigsäure zugegeben. Im Verlauf von 4 Tagen wurde das Substrat zu 3-Hydroxyphenylessigsäure demethyliert und weiter zu Homogentisinsäure hydroxyliert.

### Beispiele 6 und 7

### Herstellung von halogenierten Hydroxyphenylessigsäuren

Jeweils ein 10 l-Fermenter wurae mit einer Vorkultur (3mal 330 ml) der Mutante LU 6577 angeimpft und 3 Tage bei 30°C inkubiert, wie in Beispiel 2 beschrieben. Die Eduktkonzentration in der Vorkultur betrug 0,5 g/l. In der Hauptkultur betrug die Eduktkonzentration 5 g/l. Die Ergebnisse der Experimente sind aus Tabelle 1 ersichtlich.

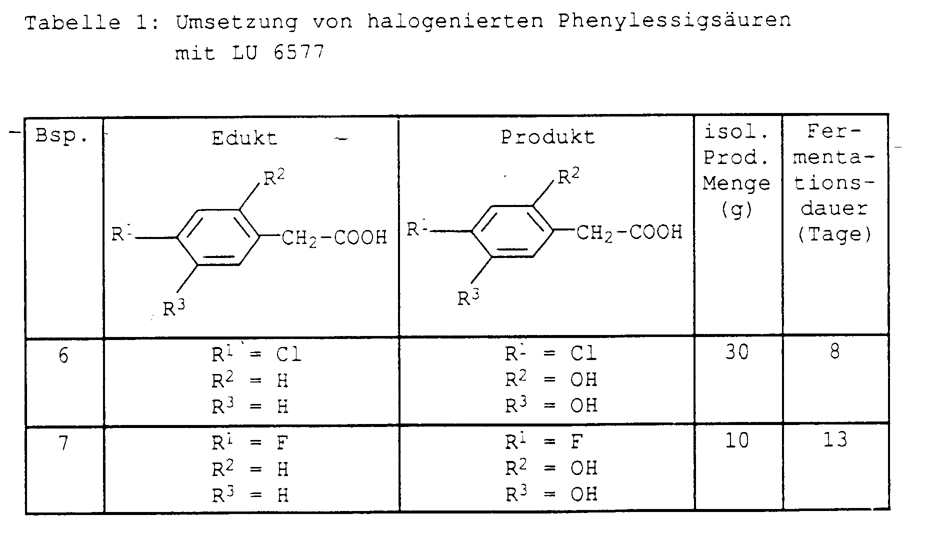

## Patentansprüche

1. Verfahren zur fermentativen Herstellung von Verbindungen der allgemeinen Formel I 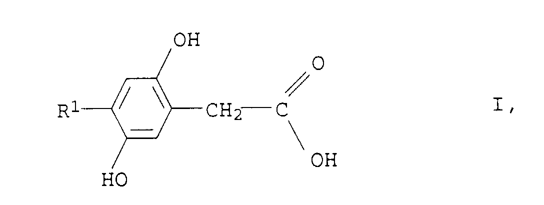 worin
R¹ ein Wasserstoff-, ein Fluor-, ein Chlor- oder ein Bromatom bedeutet,
dadurch gekennzeichnet, daß man Verbindungen der allgemeinen Formel II 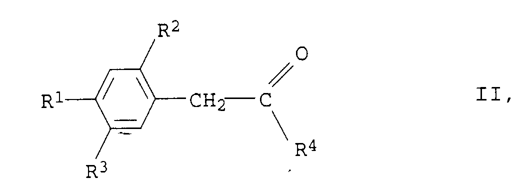 worin
R¹ die obengenannten Reste,
R², R³ unabhängig voneinander ein Wasserstoffatom oder eine Hydroxylgruppe oder eine Methoxygruppe und
R⁴ eine Hydroxylgruppe oder eine Methoxygruppe oder eine Aminogruppe bedeuten,
in Gegenwart eines Mikroorganismus der Gattung Beauveria hydroxyliert, der Verbindungen der Formel II hydroxylieren, jedoch nicht als C-Quelle verwerten kann.

2. Verfahren nach Anspruch 1, dadurch gekennzeichnet, daß als Mikroorganismus Beauveria bassiana verwendet wird.

## Claims

1. A process for the preparation, by fermentation, of a compound of the formula I 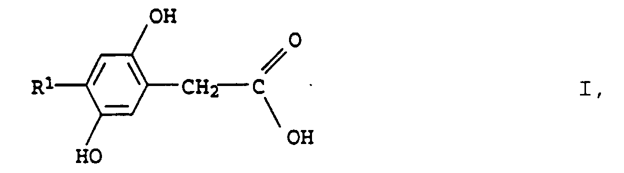 where
R¹ is hydrogen, fluorine, chlorine or bromine,
which comprises hydroxylating a compound of the formula II 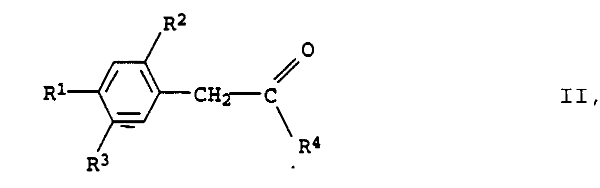 where
R¹ has the abovementioned meanings,
R² and R³ are each, independently of one another, hydrogen, hydroxyl or methoxy and
R⁴ is hydroxyl, methoxy or amino,
in the presence of a microorganism of the genus Beauveria which is able to hydroxylate, but not utilize as C source, compounds of the formula II.

2. A process as claimed in claim 1, wherein Beauveria bassiana is used as microorganism.

## Revendications

1. Procédé pour la préparation par fermentation de composés de formule générale I 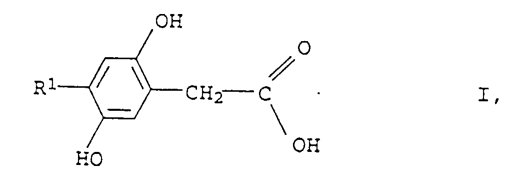 dans laquelle
R¹ représente un atome d'hydrogène, de fluor, de chlore ou de brome,
caractérisé par le fait que l'on hydroxyle des composés de formule générale II 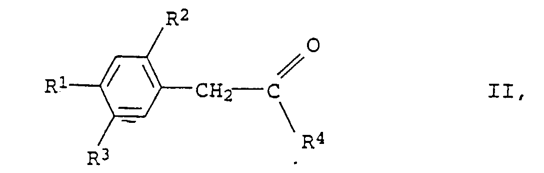 dans laquelle
R¹ a les significations indiquées ci-dessus,
R² et R³ représentent chacun, indépendamment l'un de l'autre, un atome d'hydrogène ou un groupe hydroxy ou un groupe méthoxy et
R⁴ représente un groupe hydroxy ou méthoxy ou un groupe amino,
en présence d'un microorganisme du genre Beauveria, capable d'hydroxyler les composés de formule II mais non de servir de source de carbone.

2. Procédé selon la revendication 1, caractérisé par le fait que l'on utilise, en tant que microorganisme, Beauveria bassiana.
